# EUROPÄISCHE PATENTANMELDUNG

(11) **EP 1 148 442 A2**
(43) Veröffentlichungstag der Anmeldung: **24.10.2001**
(21) Anmeldenummer: 01000110.5
(22) Anmeldetag: 11.04.2001
(51) Int. Cl.: G06T 5/00

(54) **Röntgenuntersuchungsgerät und Verfahren zur Erzeugung eines Röntgenbildes**

(30) Priorität: 20.04.2000 DE 10019955
(71) Anmelder: Philips Corporate Intellectual Property GmbH, 52064 Aachen (DE); Koninklijke Philips Electronics N.V., 5621 BA Eindhoven (NL)
(72) Erfinder: Eck, Kai c/o Philips Corp. Int. Property GmbH, 52064 Aachen (DE); Jung, Norbert,Dr. c/o Philips Corp. Int. Prop.GmbH, 52064 Aachen (DE); Meulenbrugge, Henk c/o Philips Corp.Int.Prop.GmbH, 52064 Aachen (DE)
(74) Vertreter: Volmer, Georg, Dipl.-Ing.

(57) **Zusammenfassung**

Die Erfindung betrifft ein Röntgenuntersuchungsgerät und Verfahren zur Erzeugung eines Röntgenbildes mit einer Verarbeitungseinheit (2) zur Korrektur von Bilddaten. Um Abbildungsfehler, die durch Unvollkommenheiten in der Bilderzeugungs- und -verarbeitungskette hervorgerufen werden, automatisch zu korrigieren ist Verarbeitungseinheit (2) eine Fehlerdetektionseinheit (3) zur Detektion von Bildfehlern nachgeschaltet, die anhand von Bildparametern detektierbar sind, die aus Bilddaten extrahierbar sind, die während klinischer Untersuchungen anfallen. Die Fehlerdetektionseinheit ist geeignet, in der Verarbeitungseinheit (2) angewendete Verarbeitungsparameter (15-21) in Abhängigkeit der detektierten Bildfehler zu adaptieren. Zur Detektion von Bildfehlern die insbesondere durch defekte Sensorelemente oder Pixel des Röntgendetektors hervorgerufen werden, ist eine Filtereinheit (37) vorgesehen, mittels derer in Abhängigkeit eines Schwellwertes eine Fehlertabelle für defekte Sensorelemente erstellt wird, anhand der eine Korrekturtabelle 20 in der Verarbeitungseinheit (2) erstellt wird, die auf die Bilddaten angewendet wird.

## Beschreibung

Die Erfindung betrifft ein Röntgenuntersuchungsgerät und ein Verfahren zur Erzeugung eines Röntgenbildes.

In der US 5657400 wird ein Verfahren zur Korrektur defekter Pixelwerte eines Röntgendetektors beschrieben. Es wird ein Verfahren zur Identifizierung und Korrektur defekter Pixel angegeben. Ein Korrekturcode wird für jedes als defekt identifiziertes Pixel in einer Defekttabelle gespeichert. Für die Identifizierung werden zuerst mehrere Offsetbilder akquiriert, die dann gemittelt werden. Als nächstes werden mehrere Röntgenbilder mit homogener Belichtung in einer speziellen Kalibrationsprozedur aufgenommen, die ebenfalls gemittelt werden. Diese zwei gemittelten Bilder werden voneinander subtrahiert. Anhand der Differenz werden defekte Pixel bestimmt. Die Kalibrationsprozedur wird vor der eigentlichen Röntgenaufnahme vorgenommen, ohne dass ein Patient im Röntgenstrahlengang befindlich ist.

Für die zu Diagnosezwecken erforderliche fehlerfreie Darstellung von Röntgenbildern ist es notwendig, die vom Röntgenuntersuchungsgsrät erzeugten Bilddaten zu korrigieren. Beispielsweise bewirkt die Geometrie von Röntgendetektoren Abbildungsfehler, die mittels Korrekturtabellen in der Bildverarbeitung beseitigt werden. Weiterhin können dem Röntgendetektor Offsetwerte anhaften, die ebenfalls mittels Bildverarbeitung korrigiert werden. In dieser Bildverarbeitung werden alle dem Hersteller bekannten Abbildungsfehler korrigiert, wobei die Bildverarbeitung hier nur auf Bildparametern basiert, die der Hersteller des Röntgenuntersuchungsgerätes a priori zur Verfügung stellt oder die mittels Kalibrationsprozessen derartiger Röntgenuntersuchungsgeräte gewonnen wurden.

Die Einsatzmöglichkeiten für Röntgenuntersuchungsgeräte unterschieden sich jedoch stark, so dass die Belastungen und wirkenden Einflüsse für jedes einzelne Röntgenuntersuchungsgerät unterschiedlich sind
Für die Erzeugung eines Röntgenbildes ist ein Zusammenspiel mehrerer Komponenten erforderlich, wobei jede einzelne Komponente einem Alterungsprozeß oder nichtvorhersehbaren Umwelteinflüssen oder Belastungen ausgesetzt ist. Diese nichtvorhersehbaren Einflüsse beeinträchtigen die Qualität des darzustellenden Röntgenbildes in untetschiedlicher Weise.

Dieser Art von Beeinträchtigung kann mittels periodischer Updates von Korrekturtabellen in der Bildverarbeitung abgeholfen wrden.

Die Fehler, die bei der Aufnahme von Röntgenbildern auftreten können, sind jedoch nicht zeitstabil und auch nicht unabhängig vom jeweiligen Betriebsmodus des Röntgenuntersuchungsgerätes. Mittels periodischer Updates kann eine Basisqualität der mit dem Röntgenuntersuchungsgerät aufgenommenen Röntgenbilder sichergestellt werden. Dennoch besteht zwischen den Zeitpunkten solcher periodischer Updates das Risiko einer Über- oder Unterkompensation von Bildfehlem oder Abbildungsfehlern und das Risiko des Auftretens plötzlicher Fehler bei der Aufnahme oder der Bildverarbeitung.

In den bekannten Verfahren zur Beseitigung von Abbildungsfehlern werden Bildparameter benutzt, die nicht während der klinischen Untersuchung gewonnen wurden. Eine Bewertung des vorliegenden Abbildungsfehlers wird nicht vorgenommen, ebenso wird keine entsprechende Adaptionsmethode bestimmt.

Aufgabe der Erfindung ist es deshalb ein Röntgenuntersuchungsgerät und ein Verfahren zur Erzeugung von Röntgenbildern anzugeben, bei denen Abbildungsfehler, die durch Unvollkommenheiten in der Bilderzeugungs-und -verarbeitungskette hervorgerufen werden, automatisch korrigiert werden.

Diese Aufgabe wird mit einem Röntgenuntersuchungsgerät gelöst, mit einer Röntgenstrahlungsquelle, einem Röntgendetektor mit Sensorelementen zur Umwandlung von Röntgenstrahlung in elektrische Ladungen und
mit einer Verarbeitungseinheit zur Korrektur von Bilddaten, der eine Fehlerdetektionseinheit zur Detektion von Bildfehlern nachgeschaltet ist,
die anhand von Bildparametern detektierbar sind, die aus Bilddaten extrahierbar sind, die während klinischer Untersuchungen anfallen und die geeignet ist, in der Verarbeitungseinheit angewendete Verarbeitungsparameter in Abhängigkeit der detektierten Bildfehler zu adaptieren, bei der die Fehlerdetektionseinheit zur Detektion von insbesondere durch defekte Sensorelemente verursachte Bildfehler
eine Filtereinheit zur Filterung der Bilddaten und eine Einheit zur Mittelung der gefilterten Bilddaten und eine Vergleichseinheit zum Vergleich der gefilterten und gemittelten Bilddaten mit einem Schwellwert enthält, um in Abhängigkeit des Schwellwertes eine Fehlertabelle für die Sensorelemente zu erstellen.

Um Röntgenbilder eines Patienten zu erzeugen, ist ein Röntgenuntersuchungsgerät erforderlich, welches über einen Röntgenstrahler und einen Röntgendetektor verfügt. Der Röntgendetektor wandelt die durch die unterschiedliche Dichte des Gewebes und der Knochen eines Patienten in seiner Intensität geschwächten Röntgenstrahlen in detektierbare Ladungsträger. Diese Ladungsträger werden von einer Auslesevorrichtung einer Verarbeitungseinheit zugeführt. Bis zu diesem Stadium der Bildverarbeitung ist beispielsweise nur eine generelle Verstärkung der einzelnen Bilddaten erfolgt. In der Verarbeitungseinheit wird mittels Korrekturtabellen und Interpolationsvorschriften das Rohbild in eine darstellbare Form gebracht. Die Korrekturdaten, auf denen diese Bildverarbeitung basiert, sind jedoch nicht den jeweiligen Arbeitsbedingungen des Röntgenuntersuchungsgerätes angepaßt. Fehler, die nach einer Kalibration im Röntgenstrahler oder -detektor oder in der Korrekturtabellen auftreten, werden nicht berücksichtigt.

Deshalb basiert das erfindungsgemäße Röntgenuntersuchungsgerät auf der Auswertung von klinischen Bilddaten, die unmittelbar mit dem Gerät während des Einsatzes gewonnen werden. Diese klinischen Bilddaten werden bei klinischen Untersuchungen gewonnen, ohne dass das Gerät in spezielle Kalibrationszustände versetzt wird. Durch eine kontinuierliche Detektion von Bildfehlern wird eine gleichbleibende Bildqualität erreicht.

Der Verarbeitungseinheit ist eine Fehlerdetektionseinheit nachgeschaltet, in der mittels bestimmter Tests die darzustellenden Röntgenbilder auf die mit diesen Tests auffindbaren Abbildungsfehler untersucht werden.

Dazu werden aus den klinischen Bilddaten Bildparameter extrahiert, die charakteristisch für die Qualität der zu beurteilenden Röntgenaufnahme sind. Es werden Fehlertabellen oder -matrizen berechnet, die die Position defekter Pixel oder Sensorelemente beinhalten.

Erfindungemäß wird in der Fehlerdetektionseinheit, insbesondere zur Detektion von durch defekte Sensorelementen oder Pixel des Röntgendetektors verursachte Bildfehler eine Filterung der klinischen Bilddaten vorgenommen Nachdem diese Bilddaten pixelweise gefiltert wurden, wird über mehrere Aufnahmen eine pixelweise Mittelung der gefilterten Bilddaten vorgenommen Die dabei entstehenden Bilddaten werden pixelweise mit einem Schwellwert verglichen, um zu entscheiden, ob ein entsprechender Pixelwert des betrachteten Pixels innerhalb vorgegebener Toleranzbereiche liegt oder nicht. Wenn ein Pixelwert über einem Schwellwert liegt, wird es zu den als defekt bezeichneten Pixeln zugeordnet. Die Position dieser defekten Pixel wird in einer Fehlertabelle gespeichert. Mittels dieser Fehlertabelle, wird eine Korrekturtabelle erzeugt, in der jedem defekten Pixel ein korrigierter Pixelwert zugeordnet wird. Die Korrekturtabelle wird in der Verarbeitungseinheit abgelegt, so dass in jeder folgenden Röntgenaufnahme den als defekt identifizierten Pixeln ein korrigierter Pixelwert zugewiesen wird Die Korrekturtabelle wird beispielsweise nur dann ersetzt, wenn ein außerhalb vorgegebener Toleranzgrenzen liegender Unterschied zwischen der berechneten Fehlertabelle und der vorherigen Fehlertabelle vorliegt.

Zur Filterung werden Rangordnungsfilter, insbesondere Medianfilter eingesetzt. Zu den gefilterten Bilddaten werden die investierten Originalbilddaten addiert. Von den dadurch entstehenden Zwischenbilddaten werden die Absolutwerte gebildet, die über mehrere Röntgenaufnahmen Bemittelt werden. So entsteht ein für jedes einzelne Pixel des Röntgendetektors representativer Pixelwert, der mit einem Schwellwert verglichen wird

Auf diese Art und Weise ist es möglich, auch defekte Pixel zu detektieren, die sich bei der Darstellung als Blinker zeigen, d.h. die zeitweilig zu hohe und zu niedrige Pixelwerte zeigen.

Die Medianfilterung erfolgt in der Form, dass pixelweise der Median aus Pixelwerten einer vorgebbaren Umgebung des betrachteten Pixels gebildet wird.

Je nach Genauigkeit und Anwendungsgebiet werden als Filter Rangordnungsfilter (ROF) mit variabler Kernelgröße eingesetzt. Zu dieser Art Filterung werden beispielsweise in einer quadratischen Umgebung des zu filternden Pixels alle vorkommenden Pixelwerte nach ihrem Rang geordnet, wobei bei einem Median ROF der mittlere Pixelwert der repräsentative Filterwert ist. Mit steigender Anzahl der zu betrachtenden Umgebungspixelwerte steigt der Rechenaufwand aber auch die Qualität der Filterung
Üblicherweise wird mit Kernelgrößen von 3 x 3 oder 5 x 5 Pixeln um den betrachteten Pixel gearbeitet.

Bei der erfindungsgemäßen Defekterkennung werden ausschließlich klinische Bilddaten verwendet. Durch den Patienten, der sich im Röntgenstrahlengang befindet, verändert sich die Dämpfung des gemessenen Signals für aufeinanderfolgende Röntgenaufnahmen. Um trotz dieser zeitvarianten Unterschiede der Dämpfung eine Fehlertabelle zu generieren, die vom Bildinhalt unabhängig ist, werden die Bilddaten erst nach der lokalen Filterung gleichgerichtet und gemittelt.

Optional kann man auch mehrere Patientenröntgenaufnahmen mit unterschiedlichen Inhalten erst mitteln und dann filtern. Hierbei würde die eigentliche Bildinformation geschwächt werden.

Die Fehlertabelle für defekte Pixel kann vorteilhafterweise auch Angaben über die zeitliche Gültigkeit des Pixelwertes enthalten. Dadurch wird ermöglicht, dass nach Ablauf einer vorgebbaren Zeit ein als defekt erkannter Pixel wieder als nicht defekt deklariert wird
Dadurch wird realisiert, dass ein zeitvarianter Pixeldefekt nicht für unbestimmte Zeit korrigiert wird, obwohl dieser Pixel wieder korrekte Bilddaten liefert.
Ebenso kann die aktuelle Information über defekte Pixel stärker gewichtet werden als die zuvor ermittelte Information.

Eine weitere Möglichkeit besteht darin, die gesamte Fehlertabelle nach einer gewissen Zeit zu erneuern. Ebenso ist eine Defekterkennung vor der Verarbeitungseinheit möglich, so dass alle vom Röntgenuntersuchgsgerät kommenden Bilddaten ständig auf neue defekte Pixel untersucht werden und eine Korrektur nur für die jeweils aktuelle Röntgenaufnahme durchgeführt wird

Die Defekterkennung kann, unabhängig von der Anordnung in der Bildverarbeitungskette, auf die Bilddaten jeder Röntgenaufnahme angewendet werden. Dazu ist eine hohe Rechnerleistung erforderlich. Wird die Defekterkennung nur bei jeder n-ten Aufnahme durchgeführt, kann über eine Schnittstelle auch ein normaler Personalcomputer eingesetzt werden.

Vorteilhafterweise können Informationen über systembedingte Pixelfehler bei der Auswahl der Rangordnungsfilter berücksichtigt werden, so dass bei defekten Pixelspalten oder - zeilen oder zusammenhängenden Bereichen, diese für die Fehlererkennung nicht berücksichtigt werden. Die Verwendung derartiger Information führt zum Einsatz von reduzierten Kerneln der Rangordnungsfilter. Hierbei werden die vorabbekannten defekten Pixelwerte bei der Filterung weggelassen, so dass beispielsweise bei einem 5x5 ROF nicht alle 24 Pixel, die um das zu bewirtende Pixel herum angeordnet sind, für die Defekterkennung herangezogen werden.

Jedoch muß hierbei berücksichtigt werden, dass bei zu vielen defekten Pixeln in der Umgebung des zu beurteilenden Pixel, die dann beispielsweise auf null gesetzt werden, dieser auch als falsch eingestuft wrden kann, falls keine untere Grenze in der Anzahl der zur Bewertung notwendigen Pixel gesetzt wird, die festlegt, dass bei beispielsweise weniger als drei nicht defekten Pixel zur Korrektur eine größere Umgebung gewählt werden muß.

Dies führt dann zum Einsatz von expandierten Kerneln. Diese ermöglichen die Erweiterung des Kernels bei beispielsweise vorab bekannten, möglicherweise systembedingten defekten Pixeln. Die bekannte Position der defekten Pixel wird auf null gesetzt und mit einem normalen ROF logisch-UND-verknüpft. Damit erhält man vorerst einen reduzierten ROF, der um die Anzahl fehlender Pixel, die eine zur Korrektur verwendbare Information aurweisen, ergänzt wird und erhält somit einen ROF mit expandiertem Kernel, der auf die jeweilige Defektsituation angepaßt ist. Diese ergänzten Pixel liegen dann außerhalb des normalerweise quadratisch um den zu beurteilenden Pixel angeordneten Kernel.

Vorteilhaft bei der erfindungsgemäßen Defekterkennung ist die Erkennung und Beseitigung von defekten Pixeln, ohne dass zusätzliche Röntgenaufnahmen durchgeführt werden müssen oder der Eingriff von medizinischem, oder technischen Personal erforderlich wäre. Die Defekterkennung basiert ausschließlich auf Bilddaten, die während des klinischen Einsatzes gewonnen wurden und erlaubt eine robuste und zuverlässige Defektinterpolation und Fehlerreduktion während des Betriebs des Röntgenuntersuchgsgerätes ohne dessen Verfügbarkeit zu verringern.

Da der Röntgendetektor für jeden unterschiedlichen Betriebsmodus unterschiedliche Offsetbilder aufweist, muß für jeden Modus ein Offsetbild festgestellt werden, um den Offset der einzelnen Sensorelemente bei der Darstellung des Röntgenbildes abzuziehen. Dazu werden vor jeder Röntgenaufnahme ein oder mehrere zu mittelnde Offsetbilder mittels Dunkelbildaufnahme erzeugt. Anhand dieser Dunkelbildaufnahme werden die Eigenschaften des Röntgendetektors festgestellt, die er aufweist, ohne dass er einer Röntgenstrahlung ausgesetzt wird Diese Bilddaten zur Ermittlung der Offsetbilder werden nicht während klinischer Untersuchungen gewonnen.Für die Korrektur der Offsetwerte besteht der durchzuführende Test schon in der Korrektur, d.h. immer wenn der Röntgendetektor keiner Röntgenstrahlung ausgesetzt ist, werden in festlegbaren Abständen eine Anzahl von Dunkelbildaufnahmen vorgenommen, diese werden gemittelt und eine Durchschnittsoffsettabelle wird erzeugt. Mittels derer kann die in der Verarbeitungseinheit
befindliche Korrekturvorschrift für die Offsetkorrektur je nach Auslastung des Rechners und des Röntgenuntersuchgsgerätes kontinuierlich oder periodisch geupdatet werden.

Diese beispielhaft genannten Bildparameter werden mit den entweder in der Verarbeitungseinheit abgelegten und angewendeten Verarbeitungsparametern oder mit direkt in der Fehlerdetektionseinheit abgelegten Referenzwerten verglichen, die aus den zuvor aufgenommenen Röntgenaufnahmen ermittelt wurden.
Als vorteilhaft erweist es sich, die Fehlerdetektion kontinuierlich vorzunehmen. Dadurch können kleinste Abweichungen schnell detektiert und ohne großen Zeitaufwand, möglicherweise ohne zusätzliche Strahlenbelastung korrigiert werden.

Vorteilhaft an der Erfindung ist auch, dass eine Korrektur nur dann vorgenommen wird, wenn ein Abbildungsfehler detektiert wurde. Dadurch kann Rechenkapazität und Zeit gespart werden.

Durch die in Abhängigkeit des von der Fehlerdetektionseinheit detektierten Abbildungsfehlers vorgenommene Korrektur der Verarbeitungsparameter in der Verarbeitungseinheit und/oder der Zustandsparameter des Röntgenuntersuchungsgerätes wird ein abbildungsfehlerkorrigiertes Röntgenbild wieder der Fehlerdetektionseinheit zugeführt, in der wiederum eine Fehlerdetektion vorgenommen wird. Liegen die Qualitätsbewertungskriterien innerhalb vorgebbarer Toleranzgrenzen wird das von Abbildungsfehlern befreite Röntgenbild mittels einer Ausgabeeinheit dargestellt.

Wenn in einem Röntgenbild nach einer ersten von der Fehlerdetektionseinheit ausgelösten Adaption der Verarbeitungsparameter oder Zustandsparameter ein weiterer Bildfehler oder immer noch der gleiche Bildfehler detektiert wird, ist dies ein Indiz, dass die schon vorgenommene Adaption nicht ausreichend war. Die Fehlerdetektionseinheit wird in einem derartigen Fall eine andersartige Adaption vornehmen. Falls der Betriebszustand des Röntgenuntersuchungsgerätes es erlaubt wird das Gerät beispielsweise in einen Standby-modus versetzt und eine Grundkalibration vorgenommen. Dazu werden beispielsweise Dunkelbilder aufgenommen und die Offsetkompensation neu angepaßt oder der Benutzer wird informiert, Fhantomaufnahmen vorzunehmen oder entsprechend einer Vorschrift Einstellungen an dem Röntgenuntersuchungsgerät vorzunehmen. Läßt sich der Abbildungsfehler mit den verfügbaren automatischen Adaptionsmethoden nicht beseitigen, wird ein Servicetechniker informiert. Dieser kann dann über eine Remoteverbindung den Status des Röntgenuntersuchungsgerätes abfragen und entsprechende Anpassungen der Aufnahmekriterien oder Verarbeitungsparameter vornehmen.

Die Aufgaben wird auch durch ein Verfahren zur Erzeugung von Röntgenbildern gelöst. Die Aufgabe wird auch durch ein Computerprogramm zur Korrektur von Bilddaten gelöst, bei dem insbesondere Bildfehler korrigiert werden, die von defekten Sensorelementen hervorgerufen werden, indem mittels einer Filterung klinischer Bilddaten mittels Rangordnungsfilter, einer Mittelung der gefilterten Bilddaten und in Abhängigkeit eines Schwellwertes eine Fehlertabelle für die Sensorelemente erstellt wird.

Nachfolgend wird ein Ausführungsbeispiel anhand der Zeichnungen näher erläutert.

Es zeigen:
- Fig 1: schematische Darstellung eines Röntgenuntersuchungsgerätes
- Fig. 2: Fehlerdetektion mit Pixeldefekterkennung
- Fig. 3: Diagramm zur Schwellwertbildung
- Fig 4 a-e: Rangordnungsfilter

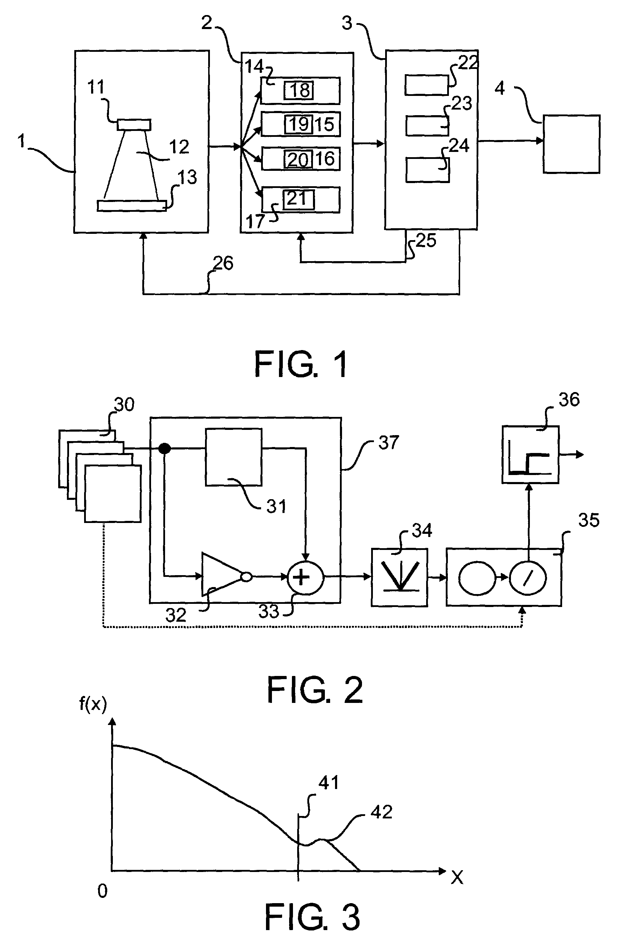

Fig 1 zeigt ein Röntgenuntersuchungsgarät mit einer Aufnahmeeinheit 1 bestehend aus Röntgenstrahler 11, Röntgenstrahlengang 12 und Röntgendetektor 13. Der Aufnahmeeinheit 1 ist eine Verarbeitungseinheit 2 nachgeschaltet, in der eine Offsetkorrektureinheit 14 mittels einer Offsetkorrekturvorschrift 18, eine Gainkorrektureinheit 15 mittels Gainkorrekturfaktor 19, eine Korrektureinheit 16 zur Korrektur defekter Pixel mittels einer Korrekturtabelle 20 und einer Einheit 17 zur Korrektur nicht linearen Verstärkerverhaltens mittels einer Look up Table (LUT) 21. Der Verarbeitungseinheit 2 ist eine Fehlerdetektionseinheit 3 nachgeschaltet, in der das von der Verarbeitungseinheit 2 korrigierte Röntgenbild auf verbleibende Bildfehler untersucht wird
Die Offsetabweichung wird mittels Dunkelbildaufnahmen korrigiert. Dabei wird bei Abweichung der neu berechneten Offsetkorrekturvorschrift 22 von der Offsetkorrekturvorschrift 18, die in der Verarbeitungseinheit 2 befindliche Offsetkorrekturvorschrift 18 ersetzt.

Die von der Verarbeitungseinheit kommenden Bilddaten werden auch in der ROF-einheit 23 auf defekte Pixel untersucht. Die ROF Einheit 23 berechnet eine Fehlertabelle, die mit der Fehlertabelle, die der Korrekturtabelle 20 in der Verarbeitungseinheit zugrunde liegt, verglichen wird. Bei Abweichung wird die Fehlertabelle und demzufolge auch die Korrekturtabelle 20 zur Korrektur defekter Pixel ersetzt.

In der Einheit 24 werden aus den Bilddaten für bestimmbare Bereiche des Röntgendetektors Histogramme über die Pixelwerte oder Grauwerte gebildet. Eine nach einer anschließenden Integration dieser Histogramme auftretende Abweichung der Kurvenverläufe indiziert nicht lineares Verstärkerverhalten zwischen beispielsweise benachbarten Bereichen des Röntgendetektors, die von unterschiedlichen Verstärkerschaltkreise ausgelesen werden. Bei Abweichung wird eine LUT berechnet, mittels derer die nicht korrekt verstärkten Pixelwerte auf die korrekt verstärkten Pixelwerte zurückgeführt werden. Diese LUT wird nur berechnet wenn zwischen den sich aus der Integration der Histogramme ergebenden Kurvenverläufen eine Abweichung vorliegt.

Wenn bei diesen Tests keine Abweichungen auftreten wird das abbildungsfehlerfreie Röntgenbild auf der Anzeigeeinheit 4 angezeigt.

Über die Verbindung 25 werden die LUTs, Fehler und Korrekturtabellen oder Offsetkorrekturvorschriften zur Verarbeitungseinheit 2 übertragen. Über die Verbindung 26 wirkt die Fehlerdetektionseinheit 3 auf die Aufnahmeeinheit 1 ein. Diese Einwirkung wird vorgenommen, falls ein Fehler trotz Update der entsprechenden Vorschrift erneut auftritt, so dass möglicherweise das Röntgenuntersuchungsgerät in einen Standby Modus versetzt werden muß oder im Röntgendetektor 13 gespeicherte Grundeinstellungen abgerufen oder initialisiert werden müssen.
Figur 2 zeigt die erfindungsgemäße Pixeldefekterkennung Die klinischen Bilddaten 30, werden einer Filtereinheit 37 zugeführt. Diese Filtereinheit 37 enthält einen Rangordnungsfilter 31, einen Inverter 32 und eine Summationseinheit 33. Die invertierten Ursprungsbilddaten werden zu den gefilterten Bilddaten addiert. Von den dadurch entstandenen Zwischenbilddaten wird jeweils in der Einheit zur Absolutwertbildung 34 der Absolutwert der Pixel gebildet. Diese Schritte werden über mehrere Frames oder Röntgenbildaufnahmen in der Einheit 35 zur Mittelung gemittelt. Dazu werden die gleichgerichteten Zuischenbilddaten durch die Anzahl der betrachteten Frames dividiert. Die dadurch entstandenen gemittelten Bilddaten werden in der Vergleichseinheit 36 mit einem Schwellwert verglichen. Alle Pixelwerte die über einem Schwellwert liegen, werden in einer Fehlertabelle gespeichert. Die in der Fehlertabelle als defekt identifizierten Pixel werden mittels der in der Verarbeitungseinheit abgelegten Korrekturtabelle 20 korrigiert und die korrigierten Pixelwerte werden in der Verarbeitungseinheit 2 auf die vom Röntgendetektor 13 kommenden Bilddaten angeziendet.

Figur 3 zeigt ein Diagramm mit dessen Hilfe der Schwellwert 41 festgelegt wird Bei einem Röntgendetektor nimmt die Anzahl der Pixel f(x), die von ihrem Idealwert abweichen kontinuierlich ab. Die defekten Pixel zeigen eine Unregelmäßigkeit 42 in diesem kontinuierlichen Verlauf. Demzufolge muß der Schwellwert kurz vor dem Abweichungswert liegen, an dem sich die defekten Pixel häufen. Eine andere Möglichkeit zur Festlegung des Schwellwertes ergibt sich, indem man hinter der Einheit 34 zur Absolutwertbildung des gefilterten und gemittelten Bildes ein Histogramm der auftretenden Grauwerte der Pixel erstellt. Eine Abweichung des normalerweise monotonen Verlaufs in diesem Histogramm deutet auf die Ansammlung defekter Pixel hin, so dass der Schwellwert auch auf diese Art und Weise ermittelt werden kann. Dabei ist es auch möglich das Histogramm kontinuierlich zu ermitteln und dadurch die Schwellwertfestlegung adaptiv vorzunehmen. Dies hat den Vorteil, das bei einer Störung, die sich auf alle Pixel gleichmäßig auswirkt und Pixelwerte erzeugt, die alle über dem zuvor manuell eingestellten Schwellwert liegen, der Schwellwert automatisch verändert wird und ein brauchbares Röntgenbild angezeigt wird, was in dem Fall mit manueller Schwellwerteinstellung nicht der Fall wäre.

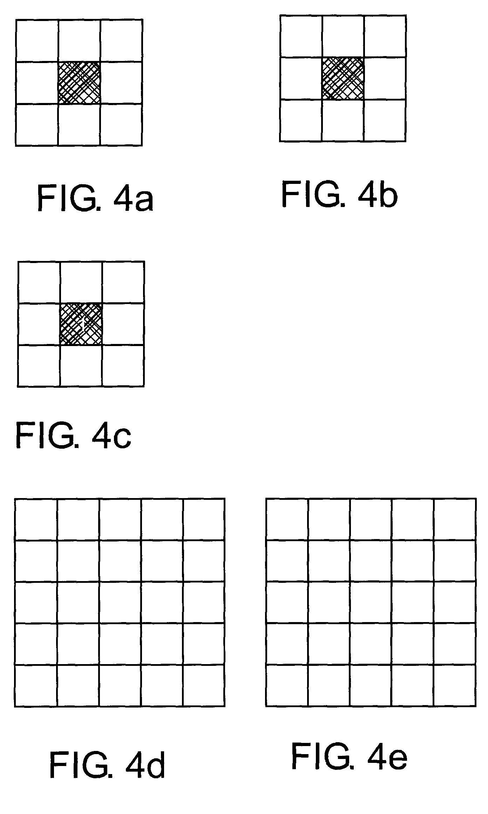

Figur 4a zeigt einen Rangordnungsfilter mit einem 3x3 Kernel. Dieser 3x3 Kernel wird bei Filterung des jeweils betrachteten Pixels angewendet, d.h. bei der Filterung eines Pixelwertes werden die zum betrachteten Pixel benachbarten Pixel mit ihren Pixelwerten in einer 3x3 Umgebung benutzt.

Figur 4 b zeigt die Position bekannter defekter Pixel, wobei das mittlere Pixel das zu beurteilende ist.

Figur 4c zeigt einen reduzierten Rangordnungsfilter, bei dem diese defekten Pixel mit ihrer bekannten Position berücksichtigt wurden. Dabei sind die defekten Pixel auf null gesetzt, so dass sie für die Filterung nicht berücksichtigt werden.

Figur 4 d zeigt die Position defekter Pixel in einer 5x5 Umgebung
Figur 4 e zeigt einen expandierten Kernel, bei dem normalerweise 9 Koeffizienten berücksichtigt werden, jedoch durch die vorab bekannten defekten Pixel keine 9 Koeffizienten mehr zur Filterung benutzt werden können. Um dies zu vermeiden, werden aus der nächsten Umgebung die fehlenden Koeffizienten aufgefüllt, damit erhält man einen ROF mit expandiertem Kernel.

## Patentansprüche

1. Röntgenuntersuchungsgerät mit
einer Röntgenstrahlungsquelle (11),
einem Röntgendetektor (13) mit Sensorelementen zur Umwandlung von Röntgenstrahlung in elektrische Ladungen und
mit einer Verarbeitungseinheit (2) zur Korrektur von Bilddaten,
und einer Fehlerdetektionseinheit (3) zur Detektion von Bildfehlern,
die anhand von Bildparametern detektierbar sind, die aus Bilddaten extrahierbar sind, die während klinischer Untersuchungen anfallen und die geeignet ist, in der Verarbeitungseinheit (2) angewendete Verarbeitungsparameter (18-21) in Abhängigkeit der detektierten Bildfehler zu adaptieren,
**dadurch gekennzeichnet,**
**dass** die Fehlerdetektionseinheit (3) zur Detektion von insbesondere durch defekte Sensorelemente verursachte Bildfehler
eine Filtereinheit (37) zur Filterung der Bilddaten
und eine Einheit (35) zur Mittelung der gefilterten Bilddaten und
eine Vergleichseinheit (36) zum Vergleich der gefilterten und gemittelten Bilddaten mit einem Schwellwert enthält, um in Abhängigkeit des Schwellwertes eine Fehlertabelle für die Sensorelemente zu erstellen.

2. Röntgenuntersuchungsgerät nach Anspruch 1,
**dadurch gekennzeichnet,**
**dass** die Fehlerdetektionseinheit (3) vorgesehen ist, dem Röntgenuntersuchungsgerät anhaftende Zustandsparameter zu adaptieren.

3. Röntgenuntersuchungsgerät nach Anspruch 1,
**dadurch gekennzeichnet,**
**dass** eine kontinuierliche Detektion vorgesehen ist.

4. Röntgenuntersuchungagerät nach Anspruch 1
**dadurch gekennzeichnet,**
**dass** die Filtereinheit (37) einen Rangordnungsfilter (31) zur Filterung der Bilddaten, einen Inverter (32) zur Invertierung Bilddaten und
eine Summationseinheit (33) zur Summierung der gefilterten und invertierten Bilddaten enthält und eine Einheit (34) zur Bildung der Absolutwerte der summierten Bilddaten vorgesehen ist.

5. Röntgenuntersuchungsgerät nach Anspruch 1,
**dadurch gekennzeichnet,**
**dass** die Fehlerdetektionseinheit (3) vorgesehen ist, bei der Detektion defekter Sensorelemente eine korrigierte Fehlertabelle der Verarbeitungseinheit (2) zuzuführen.

6. Röntgenuntersuchungsgerät nach Anspruch 1,
**dadurch gekennzeichnet,**
**dass** der Schwellwert vorgegeben ist oder adaptiv mittels Histogrammbildung der Bilddaten nach der Einheit (34) zur Absolutwertbildung festlegbar ist.

7. Röntgenuntersuchungsgerät nach Anspruch 5,
**dadurch gekennzeichnet**,
die Rangordnungsfilter (31) variable Kernel (Fig.4 b,e) aufweisen.

8. Verfahren zur Erzeugung von Röntgenbildern mit einem Röntgenuntersuchungsgerät nach einem der Ansprüche 1 bis 7.

9. Verfahren nach Anspruch 8,
**dadurch gekennzeichnet,**
**dass** die Adaption der Verarbeitungsparameter stufenweise erfolgt, wobei in einer eisten Stufe die Adaption während des Betriebes, in einer zweiten Stufe eine Adaption in einem Standby-Modus des Röntgenuntersuchungsgerätes und in einer dritten Stufe eine Adaption mittels Eingriff eines Benutzers vorgesehen ist.

10. Computerprogramm zur Korrektur von Bilddaten, insbesondere von durch defekte Sensorelemente verursachte Bildfehler, bei dem eine Filterung (37) klinischer Bilddaten mittels Rangordnungsfiltern (31),
eine Mittelung (35) der gefilterten Bilddaten und in Abhängigkeit eines Schwellwertes (36) eine Fehlertabelle für die Sensorelemente erstellt wird.
